# FASCICULE DE BREVET EUROPEEN

(11) **EP 4 590 243 B1**
(45) Date de publication et mention de la délivrance du brevet: **08.07.2026**
(21) Numéro de dépôt: 23812856.5
(22) Date de dépôt: 02.11.2023
(51) Int. Cl.: A61F 2/66, A61F 2/50

(54) **PIED PROTHÉTIQUE COMPORTANT UN ÉLÉMENT D'ACCOMPAGNEMENT ET UN ÉLÉMENT D'AMORTISSEMENT**
PROTHETISCHER FUSS MIT EINEM BEGLEIT- UND EINEM DÄMPFUNGSELEMENT
PROSTHETIC FOOT COMPRISING AN ACCOMPANYING ELEMENT AND A CUSHIONING ELEMENT

(30) Priorité: 07.11.2022 FR 2211552
(43) Date de publication de la demande: 30.07.2025
(73) Titulaire: Proteor, 21850 Saint-Apollinaire (FR)
(72) Inventeur: PENOT, Benjamin, 78280 Guyancourt (FR)
(74) Mandataire: Ipsilon
(86) Numéro de dépôt international: PCT/EP2023/080611
(87) Numéro de publication internationale: WO 2024/099882

(56) Documents cités:
- WO-A1-2021/245552
- CN-A- 114 852 212
- FR-A1- 2 658 717
- FR-A1- 3 063 889
- US-A- 5 766 264
- US-A1- 2014 336 782

## Description

### Domaine technique

La présente invention concerne, de façon générale, un pied prothétique.

L'invention porte plus particulièrement sur un pied prothétique permettant à l'utilisateur de procéder à un mouvement métatarso-phalangien de manière aisée et naturelle.

### Etat de la technique antérieure

Il est connu une solution de pied prothétique dans le document US 2014/336782 A1. Il est également connu par le brevet FR3063889 des pieds prothétiques comprenant un support de cheville permettant leur encastrement dans le tibia du patient. Ce support de cheville est lié sur sa partie inférieure à un coup de pied par l'intermédiaire d'un mécanisme.

Lesdits pieds prothétiques comportent en outre un talon et une pointe de pied configurés pour être en appui sur le sol, ainsi qu'un élément d'amortissement reliant la pointe du pied au support de cheville.

Le coup de pied est lié au talon par l'intermédiaire d'une biellette, ladite biellette étant articulée audit coup de pied et étant articulée audit talon. Cette biellette permet de transférer l'effort du talon vers le coup de pied.

Le coup de pied est lié à la pointe de pied par l'intermédiaire d'une liaison de type cardan. Ce cardan comprend une première articulation formant un premier axe et une deuxième articulation formant un deuxième axe, ledit deuxième axe étant couplé à un dispositif amortisseur de type ressort par exemple.

De tels pieds prothétiques permettent une amélioration du confort de marche et une adaptation au type de marche souhaitée par le patient.

En effet, la marche mobilise certains groupes musculaires lors de la phase d'appui au sol, permettant l'amortissement du choc du pied, et d'autres groupes musculaires lors de la phase de propulsion, permettant une restitution énergétique.

Toutefois, le système tel que décrit plus haut est complexe par le nombre de pièces et de liaisons entre les pièces et induit des frottements entre chacune des pièces.

La présente invention vise donc à proposer un pied prothétique plus simple, plus commode à utiliser, permettant l'amortissement du choc du pied lors de la phase d'appui et la restitution énergétique lors de la phase de propulsion, tout en pouvant être fabriquée de façon économique.

### Exposé de l'invention

Pour parvenir à ce résultat, la présente invention concerne un pied prothétique comportant un talon et une pointe de pied configurés pour être en appui sur le sol, un support de cheville et un élément d'amortissement comportant deux extrémités, la première extrémité étant reliée à ladite pointe de pied et la seconde extrémité étant reliée audit support de cheville. Ledit pied comporte en outre au moins une lame articulée d'une part à ladite pointe de pied et reliée d'autre part audit support de cheville, ledit élément d'amortissement étant relié audit support de cheville par l'intermédiaire de ladite lame et étant en outre relié par sa seconde extrémité audit talon, ledit élément d'amortissement étant disposé parallèlement à ladite lame.

L'invention permet à son utilisateur comportant un tel pied de procéder à un mouvement métatarso-phalangien et ainsi d'effectuer au moins un pas de manière aisée et naturelle.

En particulier, le talon et le fonctionnement de concert de la lame et de l'élément d'amortissement permettent l'amortissement du choc lors de l'entame et, lors de la propulsion, une restitution énergétique naturelle de sorte à garantir à l'utilisateur un pas naturel, similaire à celui d'un pied humain. De plus, la disposition de l'élément d'amortissement par rapport à la lame permet une répartition des efforts entre l'arrière du pied et l'avant du pied dans une position à plat du pied et donc une meilleure stabilité du pied prothétique.

Avantageusement, l'élément d'amortissement comporte au moins un ressort de traction relié d'une part à ladite pointe du pied au moyen d'une liaison de type pivot et d'autre part audit talon au moyen d'une autre liaison de type pivot, lesdites liaisons formant chacune un axe orthogonal au plan de symétrie de ladite pointe de pied.

Dans une position de dorsiflexion, le ressort est en position de repos et la lame est droite, puis en position à plat la lame est droite et s'étend parallèlement au ressort, puis en passant dans une position de flexion plantaire le ressort pivote autour de l'axe de la liaison pivot le reliant à la pointe de pied, et pivote autour de l'axe de la liaison pivot le reliant au talon. Le ressort est alors dans une position allongée, la lame est fléchie et ensemble, ils accumulent de l'énergie. Ensuite, le ressort et la lame restituent l'énergie accumulée afin d'aider l'utilisateur à lever la pointe de pied du sol de sorte à entrer en phase de propulsion.

De façon avantageuse, l'élément d'amortissement comporte un ou deux ou trois ressorts de traction.

De manière préférentielle, la lame est articulée à la pointe du pied au moyen d'une liaison pivot formant un axe orthogonal au plan de symétrie de ladite pointe de pied.

Avantageusement, la lame est en fibre de verre.

Avantageusement encore, la lame est en fibre de verre et comporte une couche de fibre de carbone.

Avantageusement encore, la lame est obtenue par injection plastique ou de polymère.

La lame se fléchie lors du mouvement métatarso-phalangien du pied prothétique, de sorte à accompagner le mouvement du pied et de sorte à permettre à l'utilisateur d'effectuer un pas de manière aisée et naturelle.

Préférentiellement, le talon présente une section en forme de U et comporte un amortisseur, ledit amortisseur étant disposé dans ladite section en forme de U dudit talon.

Avantageusement, l'amortisseur est une mousse en polyuréthane expansé.

Le talon, étant donné sa section en U, se déforme de sorte à écraser l'amortisseur ce qui amortit le choc dû à l'entrée en contact du pied prothétique avec le sol et permet de le stabiliser. Le talon accumule alors de l'énergie. Cette énergie accumulée par le talon est ensuite restituée afin de faciliter la rotation du pied prothétique, de la position de dorsiflexion à la position à plat.

Préférentiellement encore, le talon comporte au moins un connecteur mâle, en ce que la lame comporte au moins un connecteur femelle et en ce que le support de cheville comporte au moins un connecteur femelle, le connecteur mâle du talon étant configuré pour coopérer avec le connecteur femelle de la lame et avec le connecteur femelle du support de cheville.

De façon avantageuse, le pied prothétique comporte en outre un élément d'ajustement configuré pour permettre le réglage de l'angle entre ledit pied prothétique et le sol sur lequel ledit pied est en appui.

Avantageusement, l'élément d'ajustement est fixé sur la lame.

### Description des dessins

D'autres caractéristiques et avantages de l'invention apparaîtront encore à la lecture de la description qui va suivre. Celle-ci est purement illustrative et doit être lue en regard des dessins annexés sur lesquels :
[Fig.1] : la [Fig.1] représente une vue schématique en perspective d'un pied prothétique conforme à l'invention, prise de gauche, dans une position à plat, le pied prothétique comportant un élément d'amortissement, une lame, un élément d'ajustement et un support de cheville ;
[Fig.2] : la [Fig.2] représente une vue schématique, prise de dessus du pied prothétique de l'invention de la [Fig.1] ;
[Fig.3] : la [Fig.3] représente une vue schématique, prise de dessous du pied prothétique de l'invention de la [Fig.1], mais avec ici deux ressorts plutôt que trois ;
[Fig.4] : la [Fig.4] représente, de façon semblable à la [Fig.1], le pied prothétique selon l'invention mais sans la lame, sans l'élément d'ajustement et sans le support de cheville ;
[Fig.5] : la [Fig.5] représente de façon semblable à la [Fig.1] le pied prothétique selon l'invention mais sans l'élément d'ajustement et sans le support de cheville ;
[Fig.6] : la [Fig.6] représente une vue schématique en perspective du pied prothétique conforme à l'invention, prise de gauche, dans une position de flexion plantaire, le pied prothétique comportant un élément d'amortissement, une lame, un élément d'ajustement et un support de cheville ; et
[Fig.7] : la [Fig.7] représente de façon semblable à la [Fig.6] le pied prothétique selon l'invention, mais sans la lame, sans l'élément d'ajustement et sans le support de cheville.

Il faut noter que les figures exposent l'invention de manière détaillée pour mettre en oeuvre l'invention, lesdites figures pouvant bien entendu servir à mieux définir l'invention le cas échéant.

### Description des modes de réalisation

Le pied prothétique 1 selon l'invention est configuré pour permettre à son utilisateur comportant un tel pied 1 de procéder à un mouvement métatarso-phalangien.

En particulier, le pied prothétique 1 est configuré pour permettre à l'utilisateur d'effectuer au moins un pas de manière aisée et naturelle.

Pour effectuer ce pas, le pied prothétique 1 est tout d'abord dans une phase d'appui au sol, puis il entre dans une phase de propulsion, avant d'entrer de nouveau dans une phase d'appui pour effectuer le pas suivant.

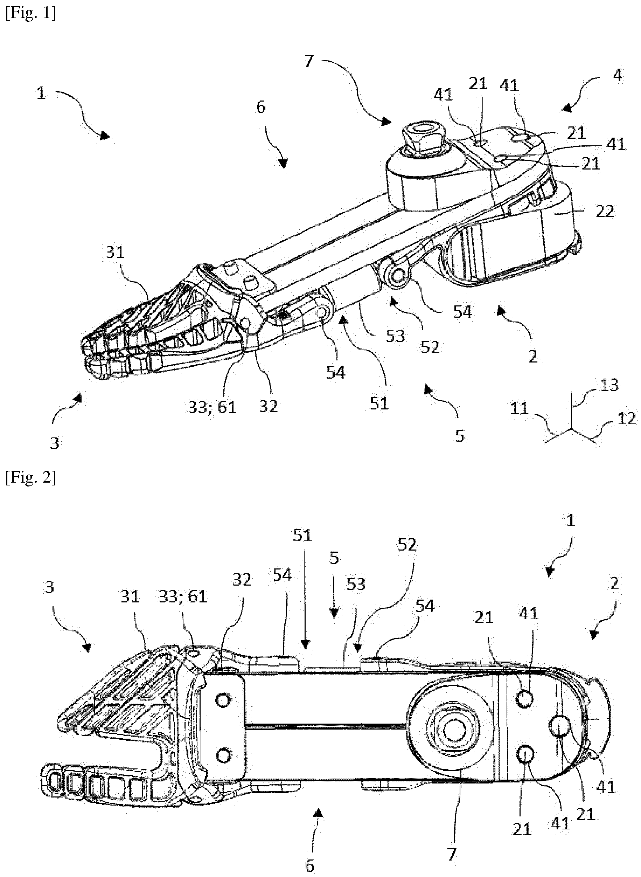

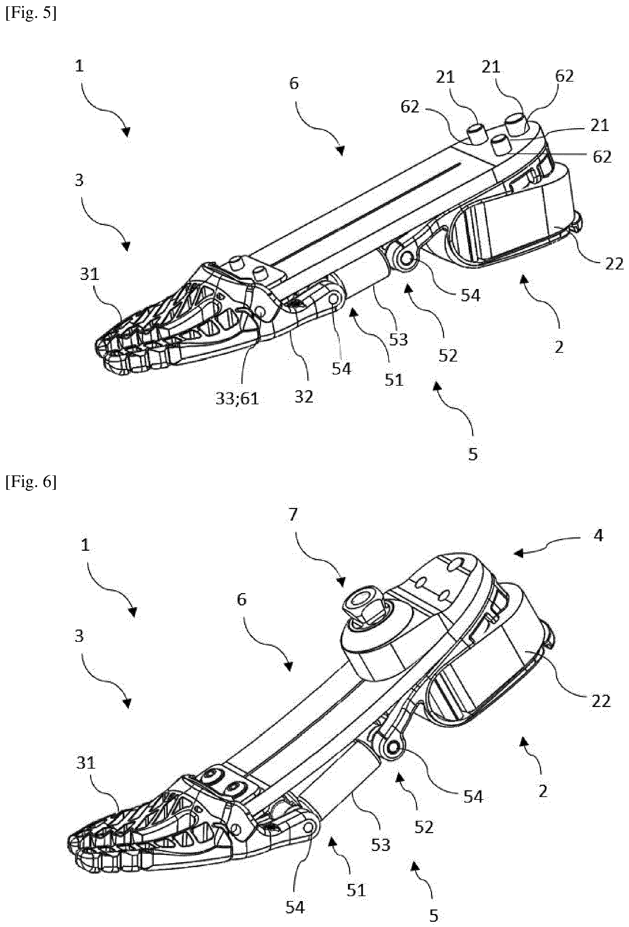

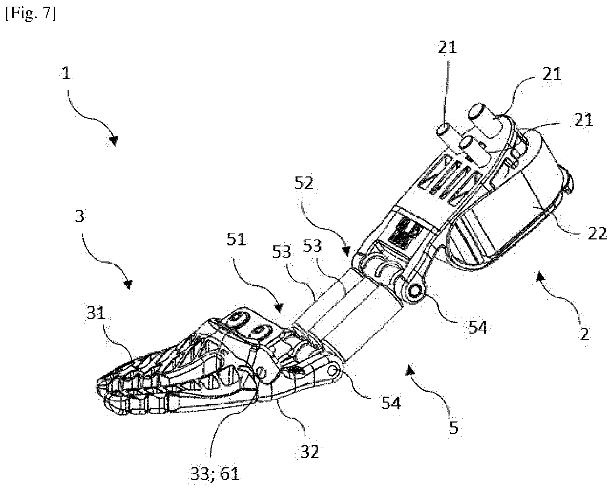

Lors de la phase d'appui au sol, le pied prothétique est tout d'abord dans une position de dorsiflexion, puis il prend une position à plat, représentée sur les figures 1 à 5, pour enfin adopter une position de flexion plantaire, représentée sur les figures 6 et 7, avant la phase de propulsion.

Le pied prothétique 1 comporte un talon 2 et une pointe de pied 3. Le talon 2 et la pointe de pied 3 sont configurés pour, dans cette position à plat, être en appui sur le sol.

Le pied prothétique 1 s'étend le long d'un axe longitudinal 11 entre le talon 2 et la pointe de pied 3. De plus, le pied prothétique 1 s'étend selon un axe transversal 12, orthogonal à l'axe longitudinal 11, et un axe vertical 13, orthogonal à l'axe longitudinal 11 et transversal 12.

Tel que cela est représenté sur la [Fig.1], la pointe de pied 3 est monobloc et comporte une première région 31 et une seconde région 32. La première région 31 correspond à l'extrémité du pied comportant les orteils et la seconde région 32 correspond à la partie pulpeuse à la base métatarse.

Avantageusement, la pointe de pied 3 et en particulier la première région 31 est ajustable. En effet, il est possible de diminuer les dimensions de la première région 31 de sorte à ajuster la pointe de pied 3 et donc le pied prothétique 1 à une pointure inférieure à la pointure initiale et/ou adapter la pointure du pied prothétique à la pointure de l'utilisateur.

Le pied prothétique 1 comporte aussi un élément d'amortissement 5 et au moins une lame 6.

Le pied prothétique 1 comporte en outre un support de cheville 4, permettant de relier le talon 2, ladite au moins une lame 6 et un élément d'ajustement 7. L'élément d'ajustement 7 est ainsi fixé sur le support de cheville 4 et présente par exemple une forme de pyramide. Il permet la connexion du pied prothétique au tibia.

L'élément d'amortissement 5 comporte deux extrémités, une première extrémité 51 reliée à la pointe de pied 3 et une deuxième extrémité 52 reliée au talon 2.

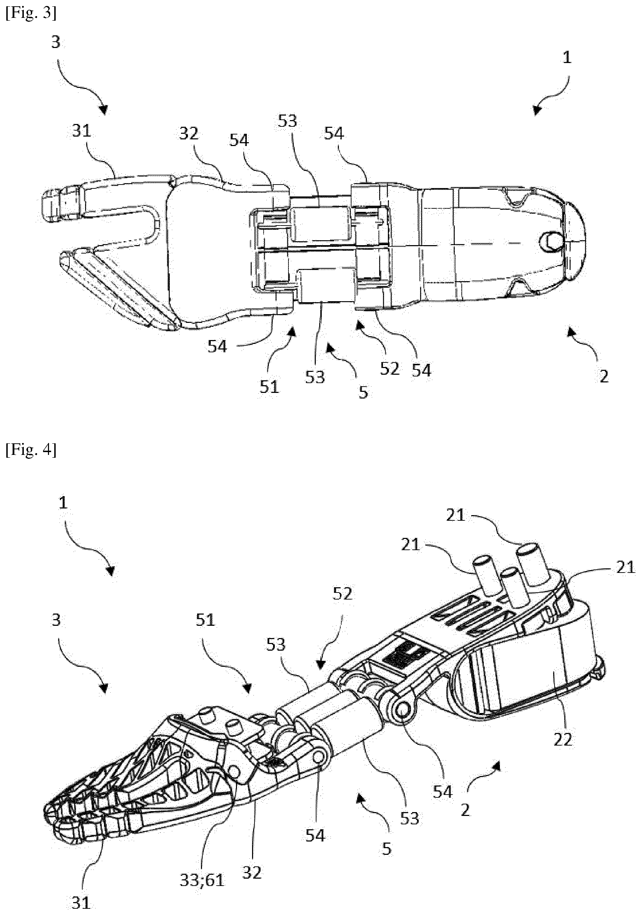

En particulier, la première extrémité 51 de l'élément d'amortissement 5 est reliée à la pointe de pied 3, cela est rendu particulièrement visible sur les figures 3 et 4. La première extrémité 51 est reliée d'une part à la pointe de pied 3 au moyen d'une liaison de type pivot 54 et la seconde extrémité 52 est reliée au talon 2 au moyen d'une autre liaison de type pivot 54. Chacune des liaisons de type pivot 54 formant un axe orthogonal au plan de symétrie de la pointe de pied 3.

L'élément d'amortissement 5 comporte au moins un ressort de traction 53.

Selon un mode de réalisation, l'élément d'amortissement 5 comporte un ressort de traction 53. Ledit ressort de traction 53 est relié d'une part à la seconde région 32 de la pointe de pied 3 au moyen de la liaison de type pivot 54 et d'autre part au talon 2 au moyen de l'autre liaison de type pivot 54.

Selon un autre mode de réalisation et tel que cela est représenté sur la [Fig.3], l'élément d'amortissement 5 peut comporter une pluralité de ressorts de traction 53, en particulier deux ressorts de traction 53. Chacun des ressorts de traction 53 est relié d'une part à la seconde région 32 de la pointe de pied 3 au moyen d'une liaison de type pivot 54 et d'autre part au talon 2 au moyen d'une autre liaison de type pivot 54.

Selon encore un autre mode de réalisation et tel que représenté sur la [Fig.4], l'élément d'amortissement 5 peut comporter trois ressorts de traction 53. Chacun des ressorts de traction 53 est relié d'une part à la seconde région 32 de la pointe de pied 3 au moyen d'une liaison de type pivot 54 et d'autre part au talon 2 au moyen d'une autre liaison de type pivot 54.

La lame 6 est d'une part articulée à la pointe de pied 3 et d'autre part reliée au support de cheville 4. Cette lame 6 est articulée à la pointe du pied 3 au moyen d'une liaison pivot 61 formant un axe orthogonal au plan de symétrie de la pointe de pied 3.

En particulier, la lame 6 est articulée à la première région 31 de la pointe de pied 3, cela est rendu particulièrement visible sur les figures 1 et 2.

Avantageusement, la lame 6 est flexible de sorte à se fléchir lors du mouvement métatarso-phalangien.

Selon un mode de réalisation de l'invention, la lame 6 peut être en fibre de verre.

Selon un autre mode de réalisation de l'invention, la lame 6 peut être en fibre de verre et comporter une couche de fibre de carbone.

Selon encore un autre mode de réalisation de l'invention, la lame 6 peut être obtenue par injection plastique ou par injection de polymère.

Selon encore un autre mode de réalisation de l'invention, la lame 6 peut être en fibre de carbone.

L'élément d'amortissement 5 et la lame 6 sont tous deux reliés par une liaison pivot respectivement 54 et 61 à la pointe de pied 3, respectivement à la seconde région 32 et à la première région 31. La liaison pivot 61 est disposée entre une extrémité libre de la pointe de pied 3 et la liaison pivot 54 de la seconde région 32 selon l'axe longitudinal 11.

Tel que cela est visible sur la [Fig.1], dans la position dite à plat, l'élément d'amortissement 5 et la lame 6 sont disposés parallèlement l'un à l'autre.

De plus, l'élément d'amortissement 5 est relié au support de cheville 4 par l'intermédiaire du talon 2 et de la lame 6. En particulier, l'élément d'amortissement 5 est relié au moyen de la liaison pivot 54 au talon 2, qui est lui-même fixé à la lame 6, elle-même fixée au support de cheville 4. Dans cette position à plat, le ressort 53 de l'élément d'amortissement 5 est en position de repos.

Le talon 2 du pied prothétique 1 présente une section en forme de U. Le talon 2 est disposé entre la lame 6 et le sol.

En outre, le talon 2 comporte un amortisseur 22 disposé dans la section en U dudit talon 2.

Avantageusement, l'amortisseur 22 est une mousse en polyuréthane expansé.

Une telle géométrie du talon, couplée à un tel amortisseur 22, permet un meilleur amortissement du choc lors de la phase d'appui, lorsque le talon 2 entre en contact avec le sol.

Le talon 2 comporte au moins un connecteur mâle 21. La lame 6 comporte au moins un connecteur femelle 62. Le support de cheville 4 comporte au moins un connecteur femelle 41.

En particulier et tel que visible sur la [Fig.4], le talon 2 comporte ici trois connecteurs mâle 21. De plus, tel que visible sur les figures 2 et 5, la lame 6 comporte ici trois connecteurs femelle 62 et le support de cheville 4 comporte ici trois connecteurs femelle 41.

Chaque connecteur mâle 21 du talon 2 est configuré pour coopérer avec un connecteur femelle 62 de la lame 6 et avec un connecteur femelle 41 du support de cheville 4.

Ainsi, le talon 2, la lame 6 et le support de cheville 4 sont solidaires les uns aux autres.

L'élément d'ajustement 7 du pied prothétique 1 est configuré pour permettre le réglage de l'angle entre le pied prothétique 1 et le sol sur lequel ledit pied 1 est en appui.

Cet élément d'ajustement 7 est fixé sur le support de cheville 4, lui-même fixé sur la lame 6.

Cet élément d'ajustement 7 est un moyen de liaison standardisé comprenant un verrouillage mécanique et permettant de régler les angles de plantiflexion et dorsiflexion du pied prothétique 1.

Les figures 6 et 7 représentent le pied prothétique 1 dans la position de flexion plantaire.

Dans cette position, seule la pointe de pied 3 est en appui sur le sol. La lame 6 a pivoté autour de l'axe de la liaison pivot 61, le support de cheville 4 fixé sur cette lame 6 a également pivoté autour de l'axe de la liaison pivot 61.

De plus, le talon 2 étant solidaire de la lame 6 a aussi pivoté autour de l'axe de la liaison 61.

L'élément d'amortissement 5 a, par sa première extrémité 51, pivoté autour de l'axe de la liaison pivot 54 le reliant à la pointe de pied 3 et a, par sa seconde extrémité 52, pivoté autour de l'axe de la liaison pivot 54 le reliant au talon 2.

Dans cette position, les ressorts 53 de l'élément d'amortissement 5 sont allongés et la lame 6 est sollicitée, c'est-à-dire s'est fléchie.

Nous allons maintenant décrire le fonctionnement du pied prothétique 1 lorsqu'un pas est effectué par l'utilisateur, à partir de la position de dorsiflexion, lorsque le talon 2 entre en contact avec le sol.

En position de dorsiflexion, le talon 2 entre en contact avec le sol et la pointe de pied 3 est vers le haut.

Dans cette position, les ressorts 53 sont dans leur position de repos et la lame 6 est droite.

Le talon 2, étant donné sa section en U, se déforme de sorte à écraser l'amortisseur 22 ce qui amortit le choc dû à l'entrée en contact du pied prothétique 1 avec le sol et permet de le stabiliser. Le talon 2 accumule alors de l'énergie.

Cette énergie accumulée par le talon 2 est ensuite restituée afin de faciliter la rotation du pied prothétique, de la position de dorsiflexion à la position à plat.

Pour ce faire, la section en U du talon 2 ainsi que l'amortisseur 22 reprennent leur position initiale.

Une fois en position à plat, le talon 2 et la pointe de pied 3 sont en appui sur le sol, la lame 6 est droite et s'étend parallèlement aux éléments d'amortissent 5. Le pied prothétique 1 à plat permet une répartition des efforts entre l'arrière du pied et l'avant du pied, c'est-à-dire entre le talon 2 et la pointe de pied 3.

En passant en position de flexion plantaire, le centre de gravité du pied prothétique 1 se déplace vers l'avant du pied, vers la pointe de pied 3.

Dans cette position de flexion plantaire, seule la pointe du pied 3 est en appui sur le sol.

L'élément d'amortissement 5 a pivoté autour de l'axe de la liaison pivot 54 le reliant à la pointe de pied 3, et pivoté autour de l'axe de la liaison pivot 54 le reliant au talon 2, respectivement par sa première extrémité 51 et sa seconde extrémité 52. Le talon 2 a également pivoté autour de l'axe de la liaison pivot 54 le reliant à l'élément d'amortissement 5. Il n'est ainsi plus en contact avec le sol. La lame 6, entre ses deux extrémités, s'est fléchie et elle pivote autour de la liaison pivot 33, de sorte à accompagner le mouvement du pied prothétique 1.

Ainsi, les ressorts 53 de l'élément d'amortissement 5 sont dans une position allongée, la lame 6 est fléchie et ensemble, ils accumulent de l'énergie.

Ensuite, les ressorts 53 et la lame 6 restituent l'énergie accumulée, afin d'aider l'utilisateur à lever la pointe de pied 3 du sol de sorte à entrer en phase de propulsion.

Suivant cette phase de propulsion, l'utilisateur va entrer de nouveau en position de dorsiflexion.

L'utilisateur peut alors entamer le pas suivant.

Le talon 2 et le fonctionnement de concert de la lame 6 et des ressorts 53 permettent l'amortissement du choc lors de l'entame et, lors de la propulsion, une restitution énergétique naturelle de sorte à garantir à l'utilisateur un pas naturel, similaire à celui d'un pied humain.

Dans des variantes non représentées :
le pied prothétique ne comporte pas une mais plusieurs lames articulées d'une part à la pointe de pied et reliées d'autre part au support de cheville de sorte à pouvoir régler la raideur du pied prothétique ;
le ou les ressorts peuvent être remplacés par un amortisseur hydraulique ou pneumatique, ou tout autre moyen permettant une adaptation du type de marche souhaitée par l'utilisateur ainsi qu'une accumulation et une restitution d'énergie telle que décrites plus haut, similaire à celle du corps humain ; le support de cheville peut comporter une partie mobile permettant à la prothèse d'être mobile selon un axe avant-arrière ou selon une pluralité d'axes, cette partie mobile peut être couplée à un amortisseur et/ou à une lame de carbone.

L'invention n'est pas limitée aux modes de réalisation précédemment décrits mais s'étend à tout mode de réalisation équivalent.

## Revendications

1. Pied prothétique comportant un talon (2) et une pointe de pied (3) configurés pour être en appui sur le sol, un support de cheville (4) et un élément d'amortissement (5) comportant deux extrémités, la première extrémité (51) étant reliée à ladite pointe de pied (3) et la seconde extrémité (52) étant reliée audit support de cheville (4), ledit pied (1) comportant en outre au moins une lame (6) articulée d'une part à ladite pointe de pied (3) et reliée d'autre part audit support de cheville (4), ledit élément d'amortissement (5) étant disposé parallèlement à ladite lame (6) **caractérisé en ce que** ledit élément d'amortissement (5) est relié audit support de cheville (4) par l'intermédiaire de ladite lame (6) et étant en outre relié par sa seconde extrémité (52) audit talon (2).

2. Pied prothétique selon la revendication 1, **caractérisé en ce que** l'élément d'amortissement (5) comporte au moins un ressort de traction (53) relié d'une part à ladite pointe du pied (3) au moyen d'une liaison de type pivot (54) et d'autre part audit talon (2) au moyen d'une autre liaison de type pivot (54), lesdites liaisons (54) formant chacune un axe orthogonal au plan de symétrie de ladite pointe de pied (3).

3. Pied prothétique selon la revendication 2, **caractérisé en ce que** l'élément d'amortissement (5) comporte un ou deux ou trois ressorts de traction (53).

4. Pied prothétique selon l'une quelconque des revendications précédentes, **caractérisé en ce que** la lame (6) est articulée à la pointe du pied (3) au moyen d'une liaison pivot (61) formant un axe orthogonal au plan de symétrie de ladite pointe de pied (3).

5. Pied prothétique selon l'une quelconque des revendications précédentes, **caractérisé en ce que** la lame (6) est en fibre de verre.

6. Pied prothétique selon l'une quelconque des revendications 1 à 4, **caractérisé en ce que** la lame (6) est en fibre de verre et comporte une couche de fibre de carbone.

7. Pied prothétique selon l'une quelconque des revendications 1 à 4, **caractérisé en ce que** la lame (6) est en plastique ou polymère.

8. Pied prothétique selon l'une quelconque des revendications précédentes, **caractérisé en ce que** le talon (2) présente une section en forme de U et comporte un amortisseur (22), ledit amortisseur (22) étant disposé dans ladite section en forme de U dudit talon (2).

9. Pied prothétique selon l'une quelconque des revendications précédentes, **caractérisé en ce que** le talon (2) comporte au moins un connecteur mâle (21), **en ce que** la lame (6) comporte au moins un connecteur femelle (62) et **en ce que** le support de cheville (4) comporte au moins un connecteur femelle (41), le connecteur mâle (21) du talon (2) étant configuré pour coopérer avec le connecteur femelle (62) de la lame (6) et avec le connecteur femelle (41) du support de cheville (4).

10. Pied prothétique selon l'une quelconque des revendications précédentes, **caractérisé en ce qu'**il comporte en outre un élément d'ajustement (7) configuré pour permettre le réglage de l'angle entre ledit pied prothétique (1) et le sol sur lequel ledit pied (1) est en appui.

11. Pied prothétique selon la revendication 10, **caractérisé en ce que** l'élément d'ajustement (7) est fixé sur la lame (6).

## Patentansprüche

1. Prothesenfuß, umfassend eine Ferse (2) und einen Vorfußbereich (3), die dazu ausgelegt sind, auf dem Boden aufzuliegen, einen Knöchelträger (4) sowie ein Dämpfungselement (5) mit zwei Enden, wobei das erste Ende (51) mit dem Vorfußbereich (3) verbunden ist und das zweite Ende (52) mit dem Knöchelträger (4) verbunden ist,
wobei der Fuß (1) ferner mindestens eine Blattfeder (6) umfasst, die einerseits mit dem Vorfußbereich (3) gelenkig verbunden ist und andererseits mit dem Knöchelträger (4) verbunden ist, wobei das Dämpfungselement (5) parallel zu der Blattfeder (6) angeordnet ist,
***dadurch gekennzeichnet, dass***
das Dämpfungselement (5) über die Blattfeder (6) mit dem Knöchelträger (4) verbunden ist und ferner mit seinem zweiten Ende (52) mit der Ferse (2) verbunden ist.

2. Prothesenfuß nach Anspruch 1, ***dadurch gekennzeichnet, dass*** das Dämpfungselement (5) mindestens eine Zugfeder (53) umfasst, die einerseits mittels einer Drehgelenkverbindung (54) mit dem Vorfußbereich (3) und andererseits mittels einer weiteren Drehgelenkverbindung (54) mit der Ferse (2) verbunden ist, wobei die Verbindungen (54) jeweils eine Achse bilden, die orthogonal zur Symmetrieebene des Vorfußbereichs (3) verläuft.

3. Prothesenfuß nach Anspruch 2, ***dadurch gekennzeichnet, dass*** das Dämpfungselement (5) eine, zwei oder drei Zugfedern (53) umfasst.

4. Prothesenfuß nach einem der vorhergehenden Ansprüche, ***dadurch gekennzeichnet, dass*** die Blattfeder (6) mittels einer Drehgelenkverbindung (61) mit dem Vorfußbereich (3) gelenkig verbunden ist, wobei die Drehgelenkverbindung (61) eine Achse bildet, die orthogonal zur Symmetrieebene des Vorfußbereichs (3) verläuft.

5. Prothesenfuß nach einem der vorhergehenden Ansprüche, ***dadurch gekennzeichnet, dass*** die Blattfeder (6) aus Glasfaser besteht.

6. Prothesenfuß nach einem der Ansprüche 1 bis 4, ***dadurch gekennzeichnet, dass*** die Blattfeder (6) aus Glasfaser besteht und eine Kohlenstofffaserschicht umfasst.

7. Prothesenfuß nach einem der Ansprüche 1 bis 4, ***dadurch gekennzeichnet, dass*** die Blattfeder (6) aus Kunststoff oder Polymer besteht.

8. Prothesenfuß nach einem der vorhergehenden Ansprüche, ***dadurch gekennzeichnet, dass*** die Ferse (2) einen U-förmigen Querschnitt aufweist und einen Stoßdämpfer (22) umfasst, wobei der Stoßdämpfer (22) in dem U-förmigen Querschnitt der Ferse (2) angeordnet ist.

9. Prothesenfuß nach einem der vorhergehenden Ansprüche, ***dadurch gekennzeichnet, dass*** die Ferse (2) mindestens einen männlichen Verbinder (21) umfasst, dass die Blattfeder (6) mindestens einen weiblichen Verbinder (62) umfasst und dass der Knöchelträger (4) mindestens einen weiblichen Verbinder (41) umfasst, wobei der männliche Verbinder (21) der Ferse (2) dazu ausgelegt ist, mit dem weiblichen Verbinder (62) der Blattfeder (6) sowie mit dem weiblichen Verbinder (41) des Knöchelträgers (4) zusammenzuwirken.

10. Prothesenfuß nach einem der vorhergehenden Ansprüche, ***dadurch gekennzeichnet, dass*** er ferner ein Einstellelement (7) umfasst, das dazu ausgelegt ist, eine Einstellung des Winkels zwischen dem Prothesenfuß (1) und dem Boden, auf dem der Fuß (1) aufliegt, zu ermöglichen.

11. Prothesenfuß nach Anspruch 10, ***dadurch gekennzeichnet, dass*** das Einstellelement (7) an der Blattfeder (6) befestigt ist.

## Claims

1. A prosthetic foot comprising a heel (2) and a toe portion (3) configured to bear against the ground, an ankle support (4), and a damping element (5) having two ends, the first end (51) being connected to said toe portion (3) and the second end (52) being connected to said ankle support (4),
said foot (1) further comprising at least one blade (6) articulated on the one hand to said toe portion (3) and connected on the other hand to said ankle support (4), said damping element (5) being arranged parallel to said blade (6),
***characterized in that***
said damping element (5) is connected to said ankle support (4) via said blade (6) and is furthermore connected by its second end (52) to said heel (2).

2. The prosthetic foot according to claim *1, **characterized in that*** the damping element (5) comprises at least one tension spring (53) connected on the one hand to said toe portion (3) by means of a pivot-type connection (54) and on the other hand to said heel (2) by means of another pivot-type connection (54), said connections (54) each forming an axis orthogonal to the plane of symmetry of said toe portion (3).

3. The prosthetic foot according to claim 2, ***characterized in that*** the damping element (5) comprises one, two, or three tension springs (53).

4. The prosthetic foot according to any one of the preceding claims, ***characterized in that*** the blade (6) is articulated to the toe portion (3) by means of a pivot connection (61) forming an axis orthogonal to the plane of symmetry of said toe portion (3).

5. The prosthetic foot according to any one of the preceding claims, ***characterized in that*** the blade (6) is made of glass fiber.

6. The prosthetic foot according to any one of claims 1 to 4, ***characterized in that*** the blade (6) is made of glass fiber and comprises a carbon fiber layer.

7. The prosthetic foot according to any one of claims 1 to 4, ***characterized in that*** the blade (6) is made of plastic or polymer.

8. The prosthetic foot according to any one of the preceding claims, ***characterized in that*** the heel (2) has a U-shaped cross-section and comprises a shock absorber (22), said shock absorber (22) being arranged within said U-shaped cross-section of said heel (2).

9. The prosthetic foot according to any one of the preceding claims, ***characterized in that*** the heel (2) comprises at least one male connector (21), **in that** the blade (6) comprises at least one female connector (62), and **in that** the ankle support (4) comprises at least one female connector (41), the male connector (21) of the heel (2) being configured to cooperate with the female connector (62) of the blade (6) and with the female connector (41) of the ankle support (4).

10. The prosthetic foot according to any one of the preceding claims, ***characterized in that*** it further comprises an adjustment element (7) configured to allow adjustment of the angle between said prosthetic foot (1) and the ground on which said foot (1) bears.

11. The prosthetic foot according to claim 10, ***characterized in that*** the adjustment element (7) is fixed to the blade (6).
